# EUROPEAN PATENT APPLICATION

(11) **EP 4 811 368 A1**
(43) Date of publication of application: **23.09.2026**
(21) Application number: 25164733.5
(22) Date of filing: 19.03.2025
(51) Int. Cl.: G16H 10/40, G16H 10/60, G16H 30/40, G16H 50/00

(54) **MEDICAL IMAGE ANALYSIS METHOD**

(71) Applicant: Koninklijke Philips N.V., 5656 AE Eindhoven (NL)
(72) Inventor: NICKISCH, Hannes, 5656 AG Eindhoven (NL); CAROLUS, Heike, 5656 AG Eindhoven (NL); HEESE, Harald Sepp, 5656 AG Eindhoven (NL)
(74) Representative: Philips Intellectual Property & Standards

(57) **Abstract**

A computer-implemented method (10) of medical image analysis for assessment of Coronary Artery Disease (CAD). The method includes receiving medical image data (62) and second data (52) related to a patient, where the second data is clinical data which is not image data. A pre-defined CAD analysis operation (16) is performed, which includes retrieving a pre-defined medical image analysis algorithm (60) configured to generate at least one stenosis severity score (66). The method generates one or more CAD findings (82) based on the medical image analysis algorithm, the received medical image data, and the received second data.

## Description

### FIELD OF THE INVENTION

The present invention relates to medical image analysis for assessment of coronary artery disease (CAD).

### BACKGROUND OF THE INVENTION

Diagnosis of coronary artery disease (CAD) is performed in part based on analysis of cardiac medical images. Traditionally, radiologists have relied on visual inspection of medical images, such as coronary computed tomography angiography (CCTA), to identify potential stenoses and evaluate the risk of CAD.

In recent years, automated image analysis algorithms have been developed to assist in CAD assessment. These algorithms can process medical images and generate quantitative measures, such as stenosis severity scores, to aid in diagnosis. While these automated tools have improved efficiency and consistency in image analysis, they typically rely solely on image data, potentially overlooking valuable clinical information that could enhance the accuracy of CAD assessment.

The integration of non-imaging clinical data, such as patient history, laboratory results, and electrocardiogram (ECG) findings, into the CAD assessment process remains a challenge. Current automated systems often lack the capability to incorporate this additional information, which may lead to incomplete or less accurate risk assessments.

### SUMMARY OF THE INVENTION

The invention is defined by the claims.

An aspect of the invention is a computer-implemented method of medical image analysis for assessment of Coronary Artery Disease (CAD). The method comprises receiving medical image data related to a patient, receiving second data related to the patient, wherein the second data is clinical data which is not image data, and performing a pre-defined CAD analysis operation. The CAD analysis operation includes retrieving a pre-defined medical image analysis algorithm for application to the medical image data, wherein the medical image analysis algorithm is configured to generate, as an output, at least one stenosis severity score associated with the input medical image data, and generating one or more CAD findings based on use of the medical image analysis algorithm, the received medical image data and the received second data.

This method allows for a comprehensive assessment of CAD by integrating both medical image data and non-image clinical data, potentially leading to more accurate and personalized diagnoses.

In the context of this disclosure, "image data" may be understood to mean any data that (directly) represents part or the whole of image, including but not limited to pixel-based or voxel-based data, stored in any format, whether raw, processed, or compressed, and derived from any medical imaging modality.

"Non-image data" may be understood to mean any data that does not (directly) represent an image, e.g. medical test data, medical reports, physiological parameter data. It may optionally include information associated with medical images, such as radiology reports or diagnostic interpretations of medical images, but does not itself include any images.

The method may further comprise a step of presenting the generated CAD analysis findings on a display.

In some embodiments, the method may comprise transforming the second data into a clinical parameter, and generating the CAD findings based on use of the clinical parameter. Optionally, the clinical parameter may be a prior risk score indicative of a prior CAD risk of the patient. Such a transformation achieves a standardization of potentially diverse types of clinical data, allowing multiple data sources to be integrated into the CAD analysis in a consistent and predictable way.

In at least one set of embodiments, the generating of the CAD findings may comprise configuring one or more parameters of the image analysis algorithm in dependence upon the second data, and subsequently applying the image analysis algorithm to the medical image data. In other words, in this set of embodiments, the second data is integrated into the CAD analysis by using it to adjust or tune parameters of the image analysis algorithm. Thus, the image analysis performed by the algorithm is fine tuned in dependence upon the second (non-image) clinical data pertaining to the patient.

By way of example, the image analysis algorithm may comprise one or more parameters which include a control point or threshold, and the configuring of the one or more parameters may comprise adjusting the control point or threshold in dependence upon the second data. This feature allows for dynamic adjustment of the algorithm sensitivity based on clinical context, potentially reducing false positives or false negatives in CAD detection.

In some embodiments, the generating of the CAD findings may comprise identifying, using the image analysis algorithm, one or more anatomical risk areas within the medical image data, and classifying a risk level of each of the identified anatomical risk areas based on use of the second data.

In some embodiments, the generating of the CAD findings may comprise identifying, using the image analysis algorithm, one or more anatomical risk areas within the medical image data, and selecting a subset of the anatomical risk areas identified by the image analysis algorithm in dependence upon the second data. This approach comprises filtering or refining an initial set of identified risk areas based on clinical context, potentially focusing attention on the most clinically relevant findings.

In some embodiments, the generating of the CAD findings may comprise generating an image representation of the identified one or more anatomical risk areas, and configuring the image representation to provide visual highlighting of the subset of anatomical risk areas. This visual representation enhances the interpretability of the results, potentially improving the efficiency of image reading and diagnosis.

In some embodiments, the generating of the CAD findings may comprise identifying, using the image analysis algorithm, one or more anatomical risk areas within the medical image data, and identifying, based on use of the second data, one or more additional anatomical risk areas, wherein the one or more additional anatomical risk areas may or may not be overlapping with the anatomical risk areas identified by the image analysis algorithm. With regards to the at least one stenosis severity score, in some embodiments, this may comprise a Coronary Artery Disease Reporting & Data System (CAD-RADS) classification. This is a standardized reporting system used in CAD assessment.

In some embodiments, the second data may include ECG data, one or more medical reports, and/or one or more laboratory results.

In some embodiments, the medical image data may comprise magnetic resonance (MR) image data and/or computed tomography (CT) image data, for example, CCTA (coronary computed tomographic angiography) image data.

A further aspect of the invention is a computer program product comprising computer program code configured, when run on a processor, to cause the processor to perform a method in accordance with any embodiment described in this disclosure or in accordance with any claim.

A further aspect of the invention is a processing device comprising one or more processors configured to perform a method in accordance with any embodiment described in this disclosure or in accordance with any claim.

A further aspect of the invention is a system comprising a diagnostic imaging system configured to output medical image data and a processing device in accordance with any embodiment or claim. The processing device is arranged to receive the medical image data.

In some embodiments, the system may include a CT imaging system and the medical image data may be Coronary Computed Tomographic Angiography (CCTA) image data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 illustrates a flowchart of a method for medical image analysis in accordance with one or more embodiments of the invention;
FIG. 2 depicts a block diagram of a system for medical image analysis in accordance with one or more embodiments of the invention;
FIG. 3 illustrates transformation of second data into a clinical parameter in accordance with one or more embodiments of the invention;
FIG. 4 illustrates an example embodiment of the invention in which one or more parameters of the medical image analysis algorithm are configured based on the second data;
FIG. 5 illustrates an example embodiment of the invention in which the second data is used to generate risk classifications for one or more identified risk areas;
FIG. 6 illustrates an example embodiment of the invention in which the second data is used to select a subset of a set of identified risk areas; and
FIG. 7 illustrates an example embodiment of the invention in which the second data is used to identify an additional set of one or more risk areas.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a method for assessment of Coronary Artery Disease (CAD) which utilizes both image data and non-image clinical data for a patient. The method comprises receiving medical image data related to a patient, along with second data that comprises clinical information not in the form of image data. The method further comprises performing a pre-defined CAD analysis operation using a medical image analysis algorithm configured to generate stenosis severity scores. CAD findings are generated based on the combined use of the image analysis algorithm, the medical image data, and the (non-image) second clinical data.

It is the recognition of the inventors that while automated image analysis algorithms have improved efficiency in CAD assessment, state of the art algorithms may result in overlooking important clinical information that could enhance diagnostic accuracy. By incorporating non-image data such as patient history, laboratory results, and ECG findings, the proposed method provides a more holistic approach to CAD assessment.

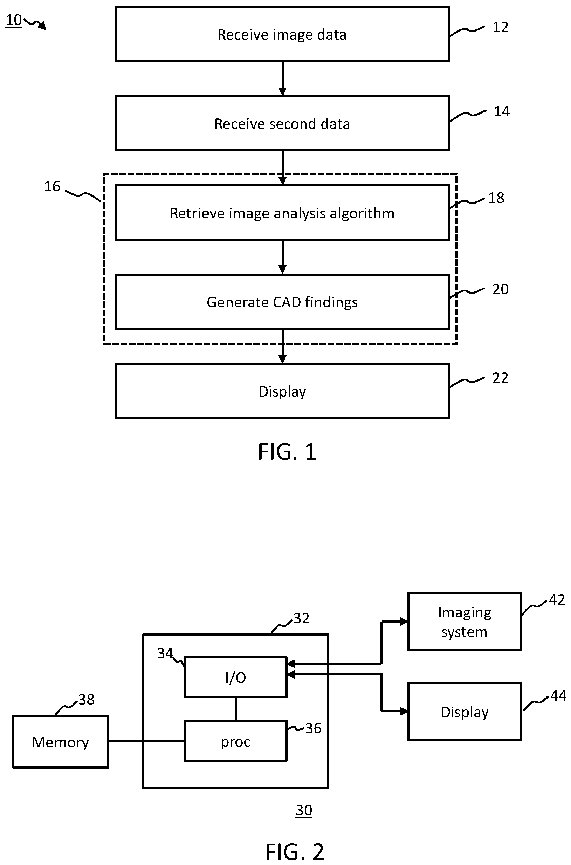

FIG. 1 illustrates a flowchart of a method 10 for medical image analysis in accordance with one or more embodiments of the invention.

The method 10 comprises receiving 12 medical image data related to a patient. By way of example, this medical image data may be Magnetic Resonance (MR) image data, Computed Tomography (CT) image data, or both. In some examples, the medical image data may be Coronary Computed Tomographic Angiography (CCTA) image data.

Following the receipt of medical image data, the method 10 further comprises receiving 14 second data related to the patient. The second data is clinical data which is not image data. By way of example, the second data may include Electrocardiogram (ECG) data, one or more medical reports, and/or one or more laboratory results.

By way of non-limiting example, the second data may include any one or more of: blood tests data, exercise tests data, body measurements, family history data, medical history (e.g. prior diseases) data, age, ethnicity, diet, medication, obesity. With regards to laboratory test data, this might include any one or more of: blood troponin test data, blood lipid test data, Pan-Immune Inflammation Value (PIV) test data, Atherogenic Index of Plasma (AIP) test data, stress test data, and/or ECG analysis data. In some embodiments, the second data may include prior PET cardiac perfusion scan data. In some embodiments, the second data may include ECG data comprising 12-lead ECG data, for example, utilizing 6 chest electrodes V1-V6 to generate 12 views at different angles of the heart.

The method 10 further comprises performing a CAD analysis operation 16. The CAD analysis operation 16 comprises retrieving 18 a pre-defined medical image analysis algorithm for application to the medical image data. This medical image analysis algorithm is configured to generate, as an output, at least one stenosis severity score associated with the input medical image data. In some examples, the stenosis severity score may comprise a Coronary Artery Disease Reporting & Data System (CAD-RADS) classification. The CAD analysis operation 16 further comprises generating 20 one or more CAD findings. The CAD findings are generated based on use of the medical image analysis algorithm, the received medical image data, and the received second data. In accordance with different embodiments, the generation of CAD findings using the image analysis algorithm, image data and non-image data can be done in different ways, and these will be outlined in the descriptions to follow, and with reference to the figures.

In some embodiments, the generating 20 the CAD findings may involve transforming the second data into a clinical parameter based on a clinical guideline.

The method 10 further comprises presenting 22 the generated CAD analysis findings on a display.

The method 10 may be implemented as a computer program product comprising computer program code configured to run on a processor. The computer program code may be configured to cause the processor to perform the method 10 as described above, or in accordance with any embodiment or claim.

A further aspect of the invention is a processing device comprising one or more processors configured to perform the method 10. The processing device may receive the medical image data from a diagnostic imaging system, for example a CT imaging system.

FIG. 2 illustrates a block diagram of an example system 30 for medical image analysis in accordance with one or more embodiments of the invention. The system 30 comprises a processing device 32 that comprises an input/output interface 34 and one or more processors 36. The processing device 32 is connected to a memory 38 for storing data and instructions. The memory 38 may store a computer program which, when run by the one or more processors 36, is configured to cause the one or more processors to perform a method in accordance with any embodiment or claim recited in this disclosure. The input/output interface 34 of the processing device 32 is connected to a diagnostic imaging system 42 that provides medical image data, and to a display device 44 for presenting analysis results.

The diagnostic imaging system 42 may be a CT imaging system configured to output medical image data, such as Coronary Computed Tomographic Angiography (CCTA) image data. The processing device 32 is arranged to receive the medical image data from the diagnostic imaging system 42 through the input/output interface 34. The processing device 32 may instead be arranged to receive the medical image data from any other source, for example from an image database or storage system, for example a PACS system.

The display device 44 is used to present algorithm results, optionally together with visual aids based on the non-image data input. These visual aids may include regional highlighting for example.

The system 30 is configured to perform the method 10 outlined previously, with reference to FIG. 1, including the steps of receiving 12 medical image data, receiving 14 second data, performing the CAD analysis operation 16, and optionally displaying 22 results. The CAD analysis operation 16 may be implemented as software instructions stored in the memory 38 and executed by the one or more processors 36.

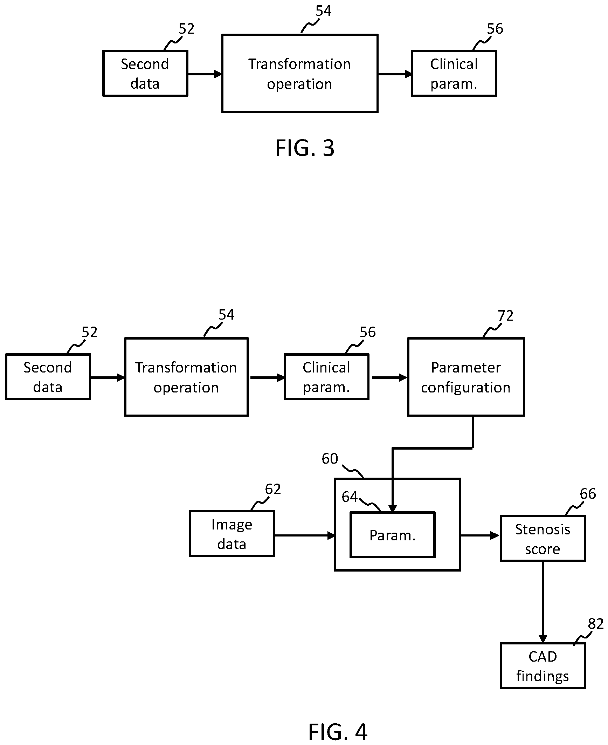

In some embodiments, the method comprises a process of transforming the second data into one or more clinical parameters in advance of performing the CAD analysis operation. This is illustrated schematically by FIG. 3.

As illustrated in FIG. 3, the method may comprise a transformation operation 54. The transformation operation receives as input the second data 52. The transformation operation 54 comprises processing the second data 52 generating as output a clinical parameter 56.

As noted above, the second data 52 may include various types of clinical data related to a patient that is not image data. For example, the second data 52 may include electrocardiogram (ECG) data, medical reports, laboratory results, and/or other relevant clinical information.

The transformation operation 54 comprises processing the second data 52 and transforming it into the clinical parameter 56. The transformation operation 54 may apply one or more pre-defined algorithms, rules, or calculations to perform the transformation.

In some examples, the clinical parameter 56 may be a prior risk score indicative of a prior Coronary Artery Disease (CAD) risk of the patient. The transformation operation 54 may analyze the second data 52 to determine factors that contribute to CAD risk, such as age, family history, cholesterol levels, blood pressure, or other relevant clinical indicators. Based on these factors, the transformation operation 54 may calculate a numerical score or risk classification that represents a prior CAD risk of the patient.

The clinical parameter 56 generated by the transformation operation 54 may be used in subsequent steps of the method 10 for medical image analysis. For example, the CAD analysis operation 16 may use the clinical parameter 56 in conjunction with the medical image data to generate the CAD findings. By transforming the second data into a clinical parameter 56, this may provide a way to integrate the second data into the CAD analysis in a standardized and reproducible way. In some embodiments, the transformation operation 54 may comprise calculating a prior risk score based on the second data 52. For instance, the transformation operation 54 may analyze factors in the second data 52 such as age, family history, cholesterol levels, blood pressure, and/or other clinical indicators to generate a numerical prior risk score. This prior risk score may then be used as the clinical parameter 56 input to the CAD analysis operation 16. The prior risk score may provide a quantitative measure of the patient's baseline CAD risk. In some cases, the transformation operation 54 may apply pre-defined risk calculation algorithms or scoring systems to convert the various clinical data points in the second data 52 into a standardized risk metric. This may allow diverse types of non-imaging clinical data to be integrated into the CAD analysis in a consistent manner across different patients. The specific algorithm used to calculate the prior risk score may be selected based on established clinical guidelines or risk assessment tools for coronary artery disease.

A method in accordance with at least one set of embodiments is outlined in more detail in FIG. 4. In this set of embodiments, the second data 52 is used to configure one or more parameters of the image analysis algorithm.

The method comprises receiving medical image data 62 and second data 52 as inputs. As illustrated, the method comprises retrieving a medical image analysis algorithm 60 configured to process the medical image data 62. The medical image analysis algorithm 60 is configured to process the medical image data 62 and generates a stenosis severity score 66 as an output.

The medical image analysis algorithm 60 may comprise one or more parameters 64 which may include a control point or threshold. In some embodiments, these one or more parameters 64 of the medical image analysis algorithm 60 are adjustable to influence how the algorithm processes the medical image data 62.

The method comprises a parameter configuration operation 72. The parameter configuration operation 72 is configured to adjust the one or more parameters 64 of the medical image analysis algorithm 60 in dependence upon the second data 52. For example, the parameter configuration operation 72 may adjust a control point or threshold of the medical image analysis algorithm 60 based on information contained in the second data 52.

In the illustrated example, the method comprises first processing the second data 52 with a transformation operation 54, to transform the second data 52 into a clinical parameter 56. The clinical parameter 56 is then used by the parameter configuration operation 72 to adjust the one or more parameters 64 of the medical image analysis algorithm 60. However, this transformation step could optionally be omitted in alternative embodiments.

After the parameter configuration operation 72 adjusts the one or more parameters 64, the medical image analysis algorithm 60 is applied to the medical image data 62. The medical image analysis algorithm 60 processes the medical image data 62 and generates a stenosis severity score 66 as an output.

The stenosis severity score 66 output from the medical image analysis algorithm 60 is used to generate CAD findings 82. The CAD findings 82 are accordingly based on both the processed medical image data 62 and the influence of the second data 52 through the parameter configuration. This example approach allows for the integration of non-image information (the second data 52) into the analysis of medical image data 62, potentially improving the accuracy and relevance of the CAD findings 82.

In some examples, the medical image analysis algorithm 60 may be implemented as a convolutional neural network (CNN) for analyzing medical images. The CNN architecture may include multiple convolutional layers for feature extraction, followed by fully connected layers for classification. For example, the CNN may have an input layer accepting medical images, five convolutional layers with ReLU activation functions, two max pooling layers, and three fully connected layers, with the final layer outputting stenosis severity scores.

The CNN may be trained on a large dataset of labeled medical images using backpropagation and stochastic gradient descent. The trained CNN may then be applied to new medical image data 62 to generate stenosis severity scores.

In some implementations, a threshold parameter 64 may be incorporated in the final fully connected layer of the CNN. This threshold may determine the cutoff for classifying an image region as having significant stenosis. The threshold parameter 64 may be adjustable based on the second data 52, allowing the sensitivity of the stenosis detection to be tuned for each patient. For example, if the second data 52 indicates the patient has high cardiovascular risk factors, the threshold may be lowered to increase sensitivity to potential stenosis.

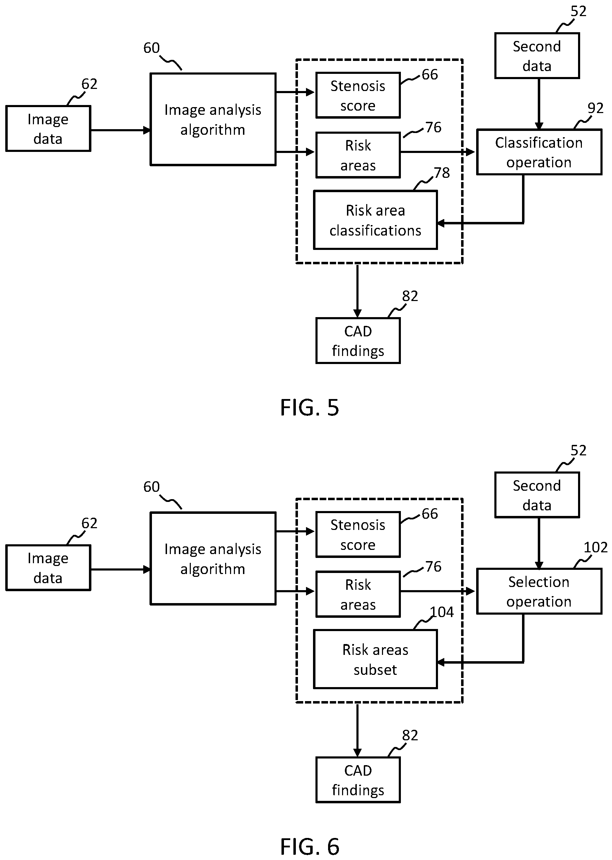

In some embodiments, the second data can be used to perform risk classification of local anatomical risk areas identified using the image data. An example is illustrated by the block diagram of FIG. 5.

The medical image analysis algorithm 60 receives the medical image data 62 as input. As noted above, in some embodiments, the medical image analysis algorithm 60 may be implemented as a convolutional neural network (CNN) for analyzing medical images.

The CNN may be trained on a dataset of labeled medical images using backpropagation and stochastic gradient descent. The trained CNN may then be applied to new medical image data 62 to generate stenosis severity scores 66.

In addition to stenosis severity scores 66, the medical image analysis algorithm 60 may be configured to identify anatomical risk areas 76 within the medical image data 62. These anatomical risk areas 76 may represent regions that exhibit characteristics associated with potential coronary artery disease, such as calcified plaques, soft plaques, or vessel narrowing.

In some embodiments, the medical image analysis algorithm 60 may output an overall risk score by combining the stenosis severity scores 66 and information about the identified anatomical risk areas 76. This overall risk score may provide a comprehensive assessment of the patient's coronary artery disease risk based on the medical image data 62.

The method in accordance with this example includes a classification operation 92. The classification operation 92 receives the second data 52 as input. The classification operation 92 uses the second data 52 to generate risk area risk level classifications 78 associated with the anatomical risk areas 76 identified by the medical image analysis algorithm 60.

The classification operation 92 may comprise analyzing the second data 52, which may include clinical information such as patient history, laboratory results, or other non-image data, to determine risk levels for each of the anatomical risk areas 76. For example, the classification operation 92 may implement a machine learning model, such as a random forest classifier or gradient boosting algorithm, trained on a large dataset of patient records with known CAD outcomes. The model may take as input various features extracted from the second data 52, such as age, gender, blood pressure, cholesterol levels, family history of heart disease, smoking status, and diabetes diagnosis. By way of illustrative example, for each anatomical risk area 76 identified by the image analysis algorithm 60, the classification model might be configured to output a risk score on a scale of 1-5, with 1 indicating very low risk and 5 indicating very high risk. The model may use different feature weightings for different anatomical regions based on known correlations between risk factors and specific coronary arteries. In some implementations, the classification operation 92 may also incorporate temporal information, such as trends in lab values over time, to refine the risk assessment. The output risk scores for each anatomical area may then be used to prioritize or highlight regions in the final CAD findings 82 presentation.

In some embodiments, the outputs from the medical image analysis algorithm 60 and the classification operation 92 may be combined to generate the CAD findings 82. The CAD findings 82 may include information about the identified anatomical risk areas 76, their associated risk area risk level classifications 78, and the stenosis severity score 66.

In some embodiments, the method may comprise using the second data to select or filter anatomical risk areas identified by the image analysis algorithm 60. An example is illustrated by the block diagram of FIG. 6.

The method comprises receiving medical image data 62 as input to a medical image analysis algorithm 60. The medical image analysis algorithm 60 processes the medical image data 62 to generate a stenosis severity score 66 and identify one or more anatomical risk areas 76.

The method further comprises receiving second clinical data 52, which is provided as input to a selection operation 102. The selection operation 102 comprises processing the anatomical risk areas 76 identified by the medical image analysis algorithm 60 using the second data 52, and to select a subset of anatomical risk areas 104 based on the second data 52.

The selection operation 102 may be configured to utilize various criteria derived from the second data 52 to determine which of the identified anatomical risk areas 76 should be included in the subset 104. For example, the selection operation 102 may prioritize risk areas that align with clinical indicators present in the second data 52, such as specific symptoms, family history, or lab results.

The outputs from the medical image analysis algorithm 60 (including the stenosis severity score 66 and the identified anatomical risk areas 76) and the selection operation 102 (the subset of anatomical risk areas 104) may be combined to generate the CAD findings 82.

This approach allows for the integration of both image data and non-image data in the CAD analysis process. By using the second data 52 to select a subset of the anatomical risk areas, the system may focus attention on the clinically most relevant findings, potentially improving the specificity and clinical utility of the CAD analysis.

In some implementations, the subset of anatomical risk areas 104 may be given higher priority in the final CAD findings 82, or may be presented with special emphasis in a visual representation of the analysis results. This may help guide clinicians to areas of particular concern based on the combination of imaging findings and non-imaging clinical data.

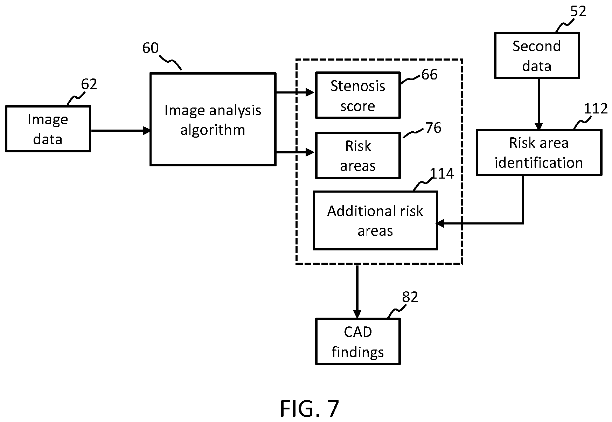

In some embodiments, the method may comprise using the second data 52 to identify or determine one or more additional anatomical risk areas to supplement risk areas identified by the image analysis algorithm 60. An example is illustrated by the block diagram of FIG. 7.

The medical image analysis algorithm 60 receives the medical image data 62 as input. The medical image analysis algorithm 60 processes the medical image data 62 to generate a stenosis severity score 66 and identify anatomical risk areas 76. The anatomical risk areas 76 may represent regions within the medical image data 62 that are indicative of potential coronary artery disease.

The method in this example includes a risk area identification operation 112. The risk area identification operation 112 receives the second data 52 as input. The risk area identification operation 112 uses the second data 52 to identify one or more additional anatomical risk areas 114 based on the second data 52.

In some implementations, the risk area identification operation 112 may employ a machine learning model, such as a random forest classifier or gradient boosting algorithm, trained on a dataset of patient records with known CAD outcomes. The model may take as input various features extracted from the second data 52, such as age, gender, blood pressure, cholesterol levels, family history of heart disease, smoking status, and/or diabetes diagnosis. For each anatomical region, the model may output a risk score, for example on a scale of 1-5, with 1 indicating very low risk and 5 indicating very high risk.

In some embodiments, the risk area identification operation 112 may use different feature weightings for different anatomical regions based on known correlations between risk factors and specific coronary arteries. The operation may also incorporate temporal information, such as trends in lab values over time, to refine the risk assessment. Regions with risk scores above a certain threshold may be identified as additional anatomical risk areas 114.

The additional anatomical risk areas 114 identified by the risk area identification operation 112 may then be combined with the anatomical risk areas 76 identified by the image analysis algorithm 60 to generate comprehensive CAD findings 82. This approach allows for the integration of both image-based and clinically-indicated risk areas, potentially providing a more complete assessment of coronary artery disease risk.

The one or more additional anatomical risk areas 114 identified by the risk area identification operation 112 may or may not be overlapping with the anatomical risk areas 76 identified by the medical image analysis algorithm 60. This means that the risk area identification operation 112 may identify risk areas that were not detected by the medical image analysis algorithm 60 alone, or may identify risk areas that coincide at least partially with those detected by the medical image analysis algorithm 60.

The outputs from the medical image analysis algorithm 60 and the risk area identification operation 112 may be combined to generate the CAD findings 82. The CAD findings 82 may include information about the identified anatomical risk areas 76, the additional anatomical risk areas 114, and the stenosis severity score 66.

In some embodiments, the method may comprise presenting the generated CAD analysis findings 82 on the display device 44. In some embodiments, the CAD findings 82 may be presented in a visual format that highlights areas of concern identified by the medical image analysis algorithm 60 and the risk area identification operation 112. The output to the display may in some examples include color-coded representations of the anatomical risk areas 76 and additional anatomical risk areas 114, along with associated risk levels and stenosis severity scores.

With regards to the medical image data 62, in some implementations, the medical image data 62 may include Magnetic Resonance (MR) image data. The medical image analysis algorithm 60 may be adapted to process MR images. Additionally or alternatively, the medical image data 62 may include computed tomography (CT) image data.

As noted above, the image analysis algorithm 60 may be configured to output a stenosis severity score 66. In some embodiments, the stenosis severity score 66 generated by the medical image analysis algorithm 60 may comprise a Coronary Artery Disease Reporting & Data System (CAD-RADS) classification. CAD-RADS is a standardized reporting system that categorizes coronary artery disease based on the degree of stenosis and other factors. The system may generate CAD-RADS scores ranging from 0 (absence of CAD) to 5 (total coronary occlusion or sub-total occlusion).

In some embodiments, the diagnostic imaging system 42 may be a CT imaging system configured to perform Coronary Computed Tomographic Angiography (CCTA). CCTA provides detailed, three-dimensional images of the coronary arteries, allowing for precise evaluation of stenosis and plaque characteristics. The medical image analysis algorithm 60 may be configured to process CCTA data, enabling accurate identification of anatomical risk areas 76 and quantification of stenosis severity.

Some particular example implementations will now be discussed by way of illustration.

In accordance with one or more examples, the second data comprises blood troponin test data. The method may comprise determining a prior risk score based on the troponin test result. For example, a higher troponin level may be indicative of higher risk and vice versa. This may be used to adjust a working point of a classifier employed by the image analysis algorithm, or may be used to identify one or more anatomical regions in the image data for visual highlighting. In some examples, a discrepancy between the second data and the output of the image analysis algorithm 60, e.g., high troponin value in conjunction with a low CAD-RADS score, may trigger a re-adjustment of the image-based risk assessment (in this scenario towards higher risk). The mechanism of re-adjustment can be a re-adjustment of the working point of a classifier to better reflect the prior knowledge, or a visual flagging of specific circumstances and conditions (e.g. via spatial highlighting) or a visual comparison of the adjusted and non-adjusted image-based risk assessment results.

In accordance with one or more examples, the second data may comprise 12-lead ECG data (using 6 chest electrodes V1-V6 to generate 12 views at different angles of the heart). This may be applicable, for example, in an acute setting such as an intensive care unit. The second data may be analyzed with an analysis algorithm to extract one or more morphological features or morphological patterns (e.g. ST segment elevation/depression or Q waves) in the ECG and their spatial distribution across particular electrodes. This can be used to classify a myocardial infarct with respect to a region of the heart involved (e.g. inferior and lateral wall), occlusion location (e.g. LCX) and age (e.g. several minutes ago). This prior knowledge (e.g. regarding ST Elevation Myocardial Infarct, i.e. STEMI) may be used in generating the final CAD findings. For example, the prior information may be used to generate a prior risk score or indicator, and this might be compared against the output of the image analysis algorithm to determine whether the two are consistent or contradictory. In some examples, the method may comprise converting detected ECG abnormalities into regional image information indicative of where the ECG abnormalities originate.

In accordance with one or more embodiments, the second data may comprise prior PET cardiac perfusion scan data. This can provide detailed information about potential perfusion defects in specific locations, for example according to the American Heart Association (AHA) standardized segmentation model. These locations may serve as the second data utilized in generating the CAD findings, for example by analyzing discrepancies and agreements with respect to an output of the image analysis algorithm 60.

While embodiments described above relate to CAD assessment, the concept may be applied to other medical domains. For example, the system may be adapted for prostate cancer risk stratification, utilizing PSA measurements, histopathology assessments, and image-based tumor-node-metastasis (TNM) staging.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application-specific integrated circuits (ASICs), and field-programmable gate arrays (FPGAs).

**In** various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (10) of medical image analysis for assessment of Coronary Artery Disease, CAD, comprising:
receiving (12) medical image data (62) related to a patient;
receiving (14) second data (52) related to the patient, wherein the second data is clinical data which is not image data;
performing a pre-defined CAD analysis operation (16) which includes:
retrieving (18) a pre-defined medical image analysis algorithm (60) for application to the medical image data, wherein the medical image analysis algorithm is configured to generate, as an output, at least one stenosis severity score (66) associated with input medical image data; and
generating (20) one or more CAD findings (82) based on use of the medical image analysis algorithm, the received medical image data and the received second data.

2. The method of claim 1,
wherein the method comprises transforming the second data into a clinical parameter, and
wherein the generating the CAD findings based on use of the second data comprises generating the CAD findings based on use of the clinical parameter, and
optionally wherein the clinical parameter is a prior risk score indicative of a prior CAD risk of the patient.

3. The method of claim 1 or 2, wherein the generating the CAD findings comprises:
configuring one or more parameters of the image analysis algorithm in dependence upon the second data; and
subsequently applying the image analysis algorithm to the medical image data.

4. The method of claim 3,
wherein the image analysis algorithm comprises one or more parameters which include a control point or threshold, and
wherein the configuring the one or more parameters of the image analysis algorithm comprises adjusting the control point or threshold in dependence upon the second data.

5. The method of any preceding claim, wherein the generating the CAD findings comprises:
identifying, using the image analysis algorithm, one or more anatomical risk areas within the medical image data, and
classifying a risk level of each of the one or more anatomical risk areas identified by the image analysis algorithm based on use of the second data.

6. The method of any preceding claim, wherein the generating the CAD findings comprises:
identifying, using the image analysis algorithm, one or more anatomical risk areas within the medical image data, and
selecting a subset of the anatomical risk areas identified by the image analysis algorithm in dependence upon the second data.

7. The method of claim 6, wherein the generating the CAD findings comprises:
generating an image representation of the identified one or more anatomical risk areas, and
configuring the image representation to provide visual highlighting of the subset of anatomical risk areas.

8. The method of any preceding claim, wherein the generating the CAD findings comprises:
identifying, using the image analysis algorithm, one or more anatomical risk areas within the medical image data, and
identifying, based on use of the second data, one or more additional anatomical risk areas, wherein the one or more additional anatomical risk areas may or may not be overlapping with the anatomical risk areas identified by the image analysis algorithm; and optionally further comprising:
generating an image representation of the identified one or more anatomical risk areas and the identified one or more additional anatomical risk areas, and
configuring the image representation to provide visual highlighting of at least the identified one or more additional anatomical risk areas.

9. The method of any preceding claim, wherein the at least one stenosis severity score comprises a Coronary Artery Disease Reporting & Data System (CAD-RADS) classification.

10. The method of any preceding claim, wherein the second data includes ECG data, one or more medical reports, and/or one or more laboratory results.

11. The method of any preceding claim, wherein the medical image data is MR image data and/or CT image data.

12. A computer program product comprising computer program code configured, when run on a processor, to cause the processor to perform a method in accordance with any preceding claim.

13. A processing device (32) comprising one or more processors (36) configured to perform a method in accordance with any of claims 1-11.

14. A system (30) comprising:
a diagnostic imaging system (42) configured to output medical image data; and
a processing device (32) in accordance with claim 13, arranged to receive the medical image data.

15. The system of claim 14, wherein the diagnostic imaging system is a CT imaging system and the medical image data is Coronary Computed Tomographic Angiography, CCTA, image data.
